(19) Europäisches Patentamt / European Patent Office / Office européen des brevets

(11) **EP 2 490 522 B1**

(12) **EUROPÄISCHE PATENTSCHRIFT**

(45) Veröffentlichungstag und Bekanntmachung des Hinweises auf die Patenterteilung:
**01.11.2017 Patentblatt 2017/44**

(51) Int Cl.:
*A01G 31/00* *(2006.01)*   *A01H 5/12* *(2006.01)*

(21) Anmeldenummer: **10768681.8**

(22) Anmeldetag: **30.09.2010**

(86) Internationale Anmeldenummer:
**PCT/EP2010/005950**

(87) Internationale Veröffentlichungsnummer:
**WO 2011/047778 (28.04.2011 Gazette 2011/17)**

(54) **VERFAHREN ZUR ERZEUGUNG VON GRUNDSTOFFEN AUS AQUATISCHEN PFLANZEN, SOWIE GRUNDSTOFFE SELBST**

METHODS FOR GENERATING BASIC MATERIALS FROM AQUATIC PLANTS, AND BASIC MATERIALS

PROCÉDÉ DE GÉNÉRATION DE MATIÈRES PREMIÈRES DE PLANTES AQUATIQUES AINSI QUE MATIÈRES PREMIÈRES

(84) Benannte Vertragsstaaten:
**AL AT BE BG CH CY CZ DE DK EE ES FI FR GB GR HR HU IE IS IT LI LT LU LV MC MK MT NL NO PL PT RO SE SI SK SM TR**

(30) Priorität: **19.10.2009 DE 102009049680**

(43) Veröffentlichungstag der Anmeldung:
**29.08.2012 Patentblatt 2012/35**

(73) Patentinhaber: **Rogmans, Maria**
**47546 Kalkar (DE)**

(72) Erfinder: **WILHELM, Hermann-Josef**
**47546 Kalkar (DE)**

(74) Vertreter: **Schmidt, Karl Michael**
**Buchenweg 65**
**47447 Moers (DE)**

(56) Entgegenhaltungen:
- BALASUBRAMANIAN P R ET AL: "RECYCLING OF BIOGAS-PLANT EFFLUENT THROUGH AQUATIC PLANT LEMNA CULTURE", BIORESOURCE TECHNOLOGY, Bd. 41, Nr. 3, 1992, Seiten 213-216, XP002621889, ISSN: 0960-8524
- CHENG JAY J ET AL: "Growing Duckweed to Recover Nutrients from Wastewaters and for Production of Fuel Ethanol and Animal Feed", CLEAN-SOIL AIR WATER, Bd. 37, Nr. 1, Januar 2009 (2009-01), Seiten 17-26, XP002621890, ISSN: 1863-0650
- TILLBERG E ET AL: "GROWTH CYCLES IN LEMNA-GIBBA CULTURES AND THEIR EFFECTS ON GROWTH RATE AND ULTRASTRUCTURE", PHYSIOLOGIA PLANTARUM, Bd. 46, Nr. 1, 1979, Seiten 5-12, XP002621891, ISSN: 0031-9317
- ÖBEK E AND HASAR H: "Role of duckweed (Lemna minor L.) harvesting in biological phosphate removal from secondary treatment effluents", FRESENIUS ENVIRONMENTAL BULLETIN, Bd. 11, Nr. 1, 2002, Seiten 27-29, XP002621892,
- SZABO S ET AL.: "Elemental flux between algae and duckweeds (Lemna gibba) during competition", ARCHIV FÜR HYDROBIOLOGIE, Bd. 146, Nr. 3, 1999, Seiten 355-367, XP002621893,
- UNDERWOOD G J C ET AL: "THE EFFECT OF VARIOUS AQUATIC BACTERIA ON THE GROWTH AND SENESCENCE OF DUCKWEED LEMNA-MINOR", JOURNAL OF APPLIED BACTERIOLOGY, Bd. 70, Nr. 3, 1991, Seiten 192-196, XP002621894, ISSN: 0021-8847
- CLATWORTHY J.N. AND HARPER J.L.: "The comparative biology of closely related species living in the same area", JOURNAL OF EXPERIMENTAL BOTANY, Bd. 13, Nr. 38, Mai 1962 (1962-05), Seiten 307-324, XP002621895,
- ASHBY E AND OXLEY T.A.: "The interction of factors in the growth of Lemna. VI. An analysis of the influence of light intensity and temperature on the assimilation rate and the rate of frond multiplication", ANNALS OF BOTANY, Bd. 49, 1935, Seiten 309-336, XP002621896,

- BALASUBRAMANIAN P R ET AL: "RECYCLING OF BIOGAS-PLANT EFFLUENT THROUGH AQUATIC PLANT LEMNA CULTURE", BIORESOURCE TECHNOLOGY, ELSEVIER BV, GB, vol. 41, no. 3, 1 January 1992 (1992-01-01), pages 213-216, XP002621889, ISSN: 0960-8524
- CHENG JAY J ET AL: "Growing Duckweed to Recover Nutrients from Wastewaters and for Production of Fuel Ethanol and Animal Feed", CLEAN - SOIL, AIR, WATER, WILEY - VCH VERLAG GMBH & CO. KGAA, DE, vol. 37, no. 1, 26 January 2009 (2009-01-26), pages 17-26, XP002621890, ISSN: 1863-0650, DOI: 10.1002/CLEN.200800210 [retrieved on 2009-01-22]
- TILLBERG E ET AL: "GROWTH CYCLES IN LEMNA-GIBBA CULTURES AND THEIR EFFECTS ON GROWTH RATE AND ULTRASTRUCTURE", PHYSIOLOGIA PLANTARUM, MUNKSGAARD INTERNATIONAL PUBLISHERS, COPENHAGEN, DK, vol. 46, no. 1, 1 January 1979 (1979-01-01), pages 5-12, XP002621891, ISSN: 0031-9317
- ÖBEK E ET AL: "Role of duckweed (Lemna minor L.) harvesting in biological phosphate removal from secondary treatment effluents", FRESENIUS ENVIRONMENTAL BULLETIN, FREISING-WEIHENSTEPHAN, DE, vol. 11, no. 1, 1 January 2002 (2002-01-01), pages 27-29, XP002621892, ISSN: 1018-4619
- SZABO S ET AL: "Elemental flux between algae and duckweeds (Lemna gibba) during competition", ARCHIV FUER HYDROBIOLOGIE, E. SCHWEIZERBART'SCHE VERLAGSBUCHHANDLUNG, STUTTGART, DE, vol. 146, no. 3, 1 October 1999 (1999-10-01), pages 355-367, XP002621893, ISSN: 0003-9136
- UNDERWOOD G J C ET AL: "THE EFFECT OF VARIOUS AQUATIC BACTERIA ON THE GROWTH AND SENESCENCE OF DUCKWEED LEMNA-MINOR", JOURNAL OF APPLIED BACTERIOLOGY, BLACKWELL PUBLISHING LTD., OXFORD, GB, vol. 70, no. 3, 1 January 1991 (1991-01-01), pages 192-196, XP002621894, ISSN: 0021-8847
- CLATWORTHY J N ET AL: "The comparative biology of closely related species living in the same area", JOURNAL OF EXPERIMENTAL BOTANY, OXFORD UNIVERSITY PRESS, GB, vol. 13, no. 38, 1 May 1962 (1962-05-01), pages 307-324, XP002621895, ISSN: 0022-0957
- ASHBY E ET AL: "The interction of factors in the growth of Lemna. VI. An analysis of the influence of light intensity and temperature on the assimilation rate and the rate of frond multiplication", ANNALS OF BOTANY, ACADEMIC PRESS, LONDON, GB, vol. 49, 1 January 1935 (1935-01-01), pages 309-336, XP002621896, ISSN: 0305-7364

Bemerkungen:

Die Akte enthält technische Angaben, die nach dem Eingang der Anmeldung eingereicht wurden und die nicht in dieser Patentschrift enthalten sind.

**Beschreibung**

Die Erfindung betrifft ein Verfahren zur Erzeugung von Grundstoffen aus aquatischen Pflanzen, gemäß Oberbegriff des Patentanspruchs 1.

Aus der Biosource Technology, 41 (1992)213-216, Recycling of Biogas-Plant Effluent through aquatic plant, P.R. Balasubramanian & Kasturi Bai, sowie aus dem Aufsatz aus Clean 2009, 37(1),17-25, Growing Duckweed to recover nutrients form Wastewater and for production of fuel ethanol and animal feed, ist der Einsatz von Wasserlinsen zur Futtermittelerzeugung oder zur Wasserreinigung bekannt. Die dabei verwendeten Kulturen sind in einer Ebene angelegt. Algenbiomasse darf als Nahrungsergänzung in Europa eingesetzt werden. Bei Algen handelt es sich aber um niedere aquatische Pflanzen. Außerdem sind sie zumeist von Wasser ganz bedeckt, bzw schwimmen ganz im Wasser. In Bezug auf die notwendige Photosynthese besteht das Problem, dass Wasser eine relativ hohe Extinktionswirkung aufweist, also nur ein Bruchteil des einfallenden Lichtes wirklich an die Algen heran lässt. Dies hat dazu geführt, dass aufwändige Beleuchtungssysteme geschaffen werden mussten.

Außerdem sind die zum Leben der Algen notwendigen biologischen Parameterbereich so eng, dass diese aufwändig geregelt werden müssen, wenn die Algen in geschlossenen Kulturen gehalten werden. Dies hat vielerorts Algenanlagen in wirtschaftliche Schwierigkeiten gebracht.

Bei der Herstellung von Futtermitteln oder Futtermittelgrundstoffen für die Tiermast, muss bei der Auswahl der Futtermittelpflanzen darauf geachtet werden, dass die entsprechende spezifische Nutzung des Viehs ein Rolle spielt. D.h. dass für die Milchproduktion bei Rindern etc bestimmte Anforderungen an das Futter gestellt werden müssen, anders als bei der Fleischproduktion.

Für Verwendungen im Bereich der Chemie im Allgemeinen und der Pharmazie und Kosmetik im Speziellen, kommt es auf inhaltsstoffliche Aspekte an, die jedoch mit der Optimierbarkeit der Biomasseerträge gekoppelt sein müssen.

Der Erfindung liegt somit die Aufgabe zugrunde, ein Verfahren, sowie Grundstoffe und Grundfuttermittel dahingehend weiterzubilden, dass große Menge an diesbezüglicher Biomasse bei überraschend guten inhaltsstofflichen Spektren erzeugt werden können.

Die gestellte Aufgabe wird bei einem Verfahren der gattungsgemäßen Art erfindungsgemäß durch die kennzeichnenden Merkmale des Anspruches 1 gelöst.

Weitere vorteilhafte Ausgestaltungen des Verfahrens sind in den abhängigen Ansprüchen 2 bis 11 angegeben.

Weitere vorteilhafte Ausgestaltungen sind in den übrigen abhängigen Ansprüchen angegeben.

Kern der verfahrensgemäßen Erfindung ist, dass die aquatischen Pflanzen selbstvermehrende Wasserlinsen sind, und ausgehend von einer Startmenge zunächst in einer Kultur vermehrt, und sodann in regelmäßigen Zeitabständen geerntet wird, derart, dass die Erntemenge maximal so groß ist, dass stets mindestens die Startmenge in der Kultur verbleibt, bis zur nächsten Beerntung.

Diese Methode ist besonders vorteilhaft bei höheren aquatischen Pflanzen, wie Wasserlinsen, Aufgrund ihres exponentiellen Zuwachses, und einer Verdopplung innerhalb von 3 bis 5 Tagen, führt das angegebene Verfahren bei den nachfolgend noch genannten aquatischen Pflanzen im Speziellen zu einem beständig hohen Massenertrag.

In weiterer vorteilhafter Ausgestaltung ist vorgesehen, dass ausgehend von einer besagten Startmenge die Vermehrung bis zum Erreichen einer Zielmenge oder Zielfläche der Kultur abgewartet wird, und eine Beerntung mit etwa dem halben Flächenmaß erfolgt, und bis zur nächsten Beerntung wieder auf das halbe Flächen- oder Gewichtsmaß durch Beerntung reduziert wird.

Weiterhin ist vorteilhaft ausgestaltet, dass nach Erreichen einer vollen Zielfläche eine tägliche Beerntung in dem Umfang durchgeführt wird, dass die beerntete Erntemenge gerade so groß ist, dass die Pflanzen wieder innerhalb eines Tages die volle Zielfläche erreichen/bedecken.

Von besonderem Vorteil ist, dass die chemischen und/oder pharmazeutischen und/oder kosmetischen Grundstoffe und/oder Futter- oder Nahrungsmittelgrundstoffe aus der Biomasse der Wasserlinse Lemna, im Besonderen der Sorte (CPVO Nr 2009/0984) Lemna Minor L Henry Blanke gewonnen wird.

Der Masseertrag dieser speziellen Sorte ist erheblich hoch, und erzielt hohe Ernteerträge.

Diese sind noch zu steigern, bei einer zusätzlichen und erhöhten Versorgung mit $CO_2$.

In weiterer vorteilhafter Ausgestaltung ist angegeben, dass die chemischen und/oder pharmazeutischen und/oder kosmetischen Grundstoffe und/oder Futter- oder Nahrungsmittelgrundstoffe aus der Biomasse der Eichhornia Crassipes, im Besonderen der Sorte (CPVO Nr 2009/0985) Eichhornia Crassipes Johann Schoofs Sen gewonnen wird. Auch diese Inhaltsstoffe sind von erheblichem Vorteil, durch die Vielzahl der Verwendungsmöglichkeiten, die dann entstehen, wenn diese Pflanze durch das erfindungsgemäße Verfahren nunmehr in großer Menge zur Verfügung steht.

In weiterer vorteilhafter Ausgestaltung ist vorgesehen, dass die chemischen und/oder pharmazeutischen und/oder kosmetischen Grundstoffe und/oder Futter- oder Nahrungsmittelgrundstoffe aus der Biomasse der Azolla, im Besonderen der Sorte (CPVO Nr 2009/0986) Azolla Avantii gewonnen wird. Hier kommt hinzu, dass Mischkulturen bspw mit Lemna derart von Vorteil sind, dass die Azolla als Stickstoffsammler die Lemna innerhalb einer Kultur im Wachstum günstig beeinflussen kann.

In weiterer vorteilhafter Ausgestaltung ist angegeben, dass die chemischen und/oder pharmazeutischen und/oder kosmetischen Grundstoffe und/oder Futter- oder Nahrungsmittelgrundstoffe aus der Biomasse einer Pflanze aus der Familie der Pistia und/oder der Silvana und/oder der Elodea gewonnen wird.

Weiterhin vorteilhaft ist, dass die chemischen und/oder pharmazeutischen und/oder kosmetischen Grundstoffe und/oder Futter- oder Nahrungsmittelgrundstoffe aus der Biomasse einer Pflanze aus der Spirodela polyrhiza und/oder der Nastrutium officiale und/oder der Ipomea aquatica gewonnen wird.

Alle diese genannten Pflanzen haben kurze Vermehrungs-oder Generationszeiten, und vermehren sich demnach exponentiell nach dem Prinzip:

$$\Delta N(t) \text{ proportional } N0$$

$$\text{D.h. } N(t) = k * e^{ct}$$

N= Anzahl der Pflanzen
T= Zeit
K und c sind konstante zum erhalt einer mathematisch konsistenten Lösung der obigen einfachen Differentialgleichung.

Die Pflanzen vermehren sich nach Impfung der Pflanzgefäße mit einer Startmasse von Pflanzen selbsttätig.

Die Zyklen, d.h. die Verdopplung der Pflanzen erfolgen bei Lemna bspw zwischen 3 bis 6 Tagen, oder noch kürzer.

Bei den anderen Pflanzen sind es ebenfalls nur wenige Tage.

Wie oben bereits ausgeführt ist es von erheblichem Vorteil, dass mehrere Pflanzen in Mischkultur kultiviert/produziert oder vermehrt werden.

In weiterer vorteilhafter Ausgestaltung ist angegeben, dass die Wasserflächen/Kulturflächen im genannten Verfahrens in einem geschlossenen Gewächshaussystem in aufgestapelten Stellagen von Wasserrinnen oder Wannen angelegt sind, wobei das Gewächshaussystem von einer $CO_2$-Quelle mit $CO_2$ gespeist wird. So können große Mengen der Biomasse auf kleinstem Raum produziert werden.

Eine weitere Ausgestaltung besteht darin, dass die Kulturflächen und/oder das besagte Gewächshaus in räumlicher Nähe zu einer Viehzuchtanlage steht, und dass ein Biomassezyklus hergstellt wird, indem erzeugte Biomasse verfüttert, und Dung und/oder CO2 und/oder Methan aus der Viehhaltung in die Kulturflächen bzw das Gewächshaus zurückgespeist werden.

Ebenso ist es bei einer kombinierten Betriebsweise von Bioreaktor und Viehstall weiterhin ausgestaltet, dass der Mist, oder Hühnermist durch Wasser abgesäuert wird auf einen pH-Wert von größer oder gleich pH 6 bis hin zu etwa pH 7. Damit steht eine ammoniumreiche Lösung im Rückführ-, d.h. Zuführwasser zu den aquatischen Pflanzenkulturen im Bioreaktor zur Verfügung. Hierzu kann auch direkt Stallluft durch Wasser geleitet werden, welches den aquatischen Pflanzen dann zugeführt wird.

Für die Erntemethode als solche, gemäß Anspruch 1 und folgende, besteht eine erheblich vorteilhafte weitere Ausgestaltung darin, die Kulturflächen zu unterteilen in lichtschwache Kulturflächen und in lichtstarke Kulturflächen. Auf den lichtschwachen Kulturflächen werden die Biomassen zunächst auf der Basis von Schwachlichtpflanzen wie Lemnacea generiert. Von diesen werden periodisch (bspw täglich) Teilmengen entnommen, bspw 20% der Flächenbedeckungen und kurzzeitig noch für eine "Nachverweilzeit" auf eine Kulturfläche mit Starklicht-bzw weniger abgeschattem Tageslicht transferiert. Dort können die Schwachlichtpflanzen nicht dauerhaft überleben, aber dennoch generieren sie dort für eine Verweilzeit von 1 bis 3 Tagen, noch mal mindestens eine Massenverdopplung, und gleichzeitig verschafft es den aquatischen Pflanzen zusätzlich noch mal eine Reifung in Bezug auf die Inhaltsstoffe. Diese Massenverdopplung verschafft dem sogenannten Folgereaktor aber auch seine ökonomische Rechtfertigung. So können sogar auch weitere Nachreaktoren in Folge eingesetzt werden, solange bis die dortigen Lichtverhältnisse auf den Kulturen 100% der Lichtmenge bzw Energie von direktem Sonnenlichteinfall entspricht. Dennoch muss auch im lichtstarken Bereich eine Abschattung vorgenommen werden, wenn die Kulturen mehr als bspw einigen 10.000 Lux ausgesetzt sind.

Wichtig ist hierbei folgender Zusammenhang:

Im Hauptreaktor herrschen Schwachlichtbedingungen. D.h. dort sind wie oben beschrieben bis zu hundert Etagen von Kulturflächen aufgestapelt und es kommt zu einer Verteilung der verfügbaren Lichtmenge auf alle Etagen, d.h. es werden zum Teil extreme Schwachlichtbedingungen eingestellt, bei der die genannte Biomasse der Lemnacea immer noch sich mixotroph ernährt stark potenziert.

Der Nachreaktor hat erheblich mehr Licht pro Kulturfläche, die man dadurch erreicht, dass man entweder keine Etagenkulturen anlegt, oder aber nur eine geringere Zahl als im Hauptreaktor.

Durch diese kurzzeitige Stimulation der Lemnacea kommt es erneut temporär zu einem Wachstumsschub, der auf Dauer aber degressiv enden würde, weil die Lemnacea NICHT Starklichtauglich ist. Doch kurzfristig hält sie dies aus, und beantwortet dies mit einem temporären Wachstumsschub.

Dieser wird ausgenutzt, umd die im Hauptreaktor erhaltene Pflanzenmasse noch ein letztes Mal zumindest zu verdoppeln.

Insbesondere in Bezug auf spezielle neue Züchtungen von Wasserlinsen basiert dies auf folgendem, während der Entwicklung verifizierten Zusammenhang. Bei der Kulturpflanze Lemnacea (Wasserlinse) handelt es sich schon von Natur her um Schwachlichtpflanzen. Hierbei werden zudem spezielle neue gezüchtete Wasserlinsensorten eingesetzt.

Nämlich

CPVO 2009/0984 Lemnacea minor HENRY BLANKE und/oder
CPVO 2010/0854 Lemnacea minor HENRY Vitesse und/oder
CPVO 2010/0857 Lemnacea minor HENRY Legato und/oder
CPVO 2010/0855 Lemnacea minor HENRY DaCapo und/oder
CPVO 2010/0856 Lemnacea minor HENRY Forte und/oder
CPVO 2010/0858 Lemnacea minor HENRY Maria und/oder
CPVO 2010/0859 Lemnacea minor HENRY Josef und/oder
CPVO 2010/0936 Spirodela HENRY Big Mama

Diese sind Züchtungen die abschließend final auf Schwachlichtauglichkeit gezüchtet wurden. Im Ergebnis führte dies zu aquatischen Pflanzen mit extrem hohem Photosynthese-Wirkungsgrad, deren energetische Versorgung somit in hohem Maße nicht mehr nur phototroph, sondern mixotroph ist.

Dies führt dazu, dass diese Wasserlinsen hohe Biomasseerträge bei vergleichsweise geringem Lichtbedarf generieren. Diese können dann in den genannten ersten (lichtschwachen) Kulturflächen zwischen den Etagen angelegt werden. Weiterhin hat sich gezeigt, dass wenn man die von den ersten Kulturflächen dann geernteten Lemnacea nochmals für mindestens oder etwa einen weiteren Tag in einen lichtstarken Bereich verbringt, dort nochmals final eine Verdopplung der Biomasse erzielt wird.

Da man von den ersten Kulturflächen nur täglich einen Teil, ca 15% bis 35% der Lemnacea-Kulturflächen erntet, damit die Population der Kultur nicht zusammenbricht. Dabei folgt man dem in der Biophysik angewendeten mathematischen Grundsatz dass der effektive Zuwachs an Masse immer proportional zur verbleibenden Masse ist.

$dM$ proportional $M_0$

Aufgelöst führt diese Differentialgleichung zum natürlichen exponentiellen Zellwachstumsverhalten,

$$dM = c * M_0$$

oder aufgelöst

Integral über $1/M\ dM$

Ergibt als Lösung:

$$M(t) = C_1 * exp(C_2 * t)$$

was auch hier für kleine Individuen (wie Wasserlinsen) in großen Populationen relevant ist. Bei überschaubaren Populationen kann man auch guten Gewissens einen quadratischen Ansatz machen, weil die Populationsrate auch noch von der Temperatur und der eingangs schon erwähnten Selbsthemmung des Wachstums nahe einer empirischen und sortenabhängig beobachtbaren Grenzdichte abhängig ist.

Um also genügend Restbiomasse ($M_0$) in der ersten Kultur zu belassen, in welcher die Verdopplungszeit 1 bis 3 Tage ist, und deshalb dort nur etwa 20 bis 40% täglich abgezogen wird, brauch man für die zweite Kulturfläche nur einen Bruchteil der Kulturfläche, wie für die erste Kulturfläche. So ist auch in etwa das Verhältnis der ersten Kulturfläche zur zweiten Kulturfläche zu wählen, und bei der erfindungsgemäßen Konstruktion ergibt sich dies auch genau so. Von der zweiten Kulturfläche wird dann immer ALLES abgerntet, weil dort ja kein $M_0$ zurückbleiben muss, aus dem Grund weil ja wieder Pflanzen von der ersten Kulturfläche in die zweite Kulturfläche transferiert werden.

Alternativ oder zusätzlich zum Anlegen der zweiten Kulturfläche im Innen- oder Lichthof der Einrichtung ist auch ausgestaltbar, dass die Anordnung der zweiten Kulturflächen alternativ oder zusätzlich zu Anspruch 17 im vorderen, lichtstarken Bereich auf den Etagen angelegt sind, und dass die ersten Kulturflächen in lichtschwachen Bereichen der Etagenanordnung angelegt sind.

Im Hinblick auf das erfindungsgemäßen Verfahren ist angegeben, dass die Kulturflächen in einer Amphitheaterförmigen, um einen inneren Lichthof rund oder elliptisch herum angelegten Etagenanordnung angelegt werden, und dass unterteilt wird in lichtschwache Areale, die zwischen den Etagen angeordnet sind, und lichtstarke Areale, die in den lichtstarken Arealen der Einrichtung platziert sind, wobei die Kulturen aus schwachlichttauglichen aquatischen Pflanzen bestehen, wie bspw Lemnacea, und zunächst zur selbsttätigen vegetativen Vermehrung in den lichtschwachen Arealen kultuviert werden, die die ersten Kulturflächen darstellen, und von dort aus dann weg in eine zweite Kulturfläche für eine zweite Verweilzeit t2, d.h. dort in die lichtstarken zweiten Areale temporär verschoben werden, von dort aus sie dann final geerntet werden.

Dieses Verfahren implementiert somit die oben beschriebene Methode noch mal zu mindestens einer finalen Verdopplung der resultierenden Biomasse.

Mit anderen Worten, die ersten Kulturflächen sind lichtschwach und sind im Hauptreaktor angelegt, während dem die lichtstarken "Nachverdopplungskulturen" in einem Nachreaktor liegen, bei denen die Kulturen jeweils mehr Licht, als im Hauptreaktor erhalten.

Da im Hauptreaktor nur Teilbeträge der Kulturflächen beerntet werden, benötigt der Nachreaktor auch nur einen Bruchteil der Kulturflächen zur Nachvermehrung als im Hauptreaktor.

Dies kann durch unterschiedliche Größe erfolgen oder aber, was sinnvoller wäre, durch ähnliche Größen aber unterschiedliche Etagendichten (Stellagendichten).

So heisst dies nun konkret, dass die gesamten aquatischen Kulturflächen sich in mindestens zwei Bioreaktoren verteilen, und zwar in der Art, dass in einem als Hauptreaktor bezeichneten ersten Reaktor eine vorgegebene erste Stellagen- oder Etagendichte (D1) für die übereinander gestapelten aquatischen Kulturen vorgesehen ist, derart, dass sich eine effektive Beleuchtungsstärke (L1) der aquatischen Flächen unter Schwachlichtbedingungen ergibt, und die Biomasse dort auf die besagte Weise vermehrt, und dass die dort periodisch erntbare Biomasse von dem Hauptreaktor in einen Nachreaktor für einen oder wenige Tage transferiert wird, wobei im Nachreaktor eine effektive Beleuchtungs- oder Ausleuchtungsstärke D2, mit D2 größer als D1, eingestellt ist, in der die dort hinein transferierten aquatischen Pflanzen noch eine weitere Massenvermehrung unter Starklichtbedingungen erhält, bevor sie final geerntet werden.

Als Beispielwerte für eine geringe Beleuchtungsstärken unter Schwachlichtbedingungen wie sie im Hauptreaktor auf den dortigen Kulturflächen vorliegen sind 100 bis 1000 Lux anzusehen.
Für die Starklichtbedingungen im Nachreaktor werden Beleuchtungsstärken von mehreren 1000 bis 50.000 Lux angesehen und eingestellt. 50.000 Lux sind jedoch so grenzwertig, dass die genannten Schwachlichtpflanzen dort tatsächlich nur einen bis wenige Tage kultiviert werden dürfen.

Bei einer dauerhaften Kultivierung bei diesen Starklichtbedingungen kommt es zur Bildung eines viellagigen System von Wasserlinsen, wobei die oberen sich opfern um die darunter liegenden zu schützen.

Weiterhin ist ausgestaltet, dass im Nachreaktor die Wassertiefe der dortigen aquatischen Kulturflächen größer ist als im Hauptreaktor.

Dies hat zur Folge, dass ein Wasseraustausch bei größerer Tiefe aus biologischen Gründen nur in sehr großen Zeitabständen (wöchentlich oder nur alle 4 oder mehr Wochen) durchgeführt werden muss. Der Nachreaktor ist dadurch sehr wirschaftlich zu betreiben.

Dies hat dann zur Folge, dass im Hauptreaktor die Wassermengen der Kulturflächen häufiger mit Frischwasser oder gedüngtem Wasser gespült werden, als im Nachreaktor.

In vorteilhafter Ausgestaltung ist angegeben, dass das Grundfuttermittel mindestens aus einer der in den Ansprüchen 4 bis 17 verwendeten Biomassen der besagten Pflanzen besteht. Deren diesbezügliche Inhaltsstoffspektren sind in der Beschreibung noch weiter ausgeführt.

Für die Herstellung von Futtermitteln oder Grundfuttermitteln kann es von Vorteil sein, dass die verwendete Biomasse siliert ist. Dabei bilden sich durch Reaktionen bspw mit Milchsäure zum Teil weitere wertvolle Inhaltsstoffe.

In Bezug auf eine Grundfuttermittel besteht dieses mindestens aus einer verwendeten Biomassen der besagten Pflanzen besteht, d.h. somit als Zugabe zu Futtermittel oder auch insgesamt aus dieser Biomasse.

Besonders vorteilhafte Grundstoffe ergeben sich für pharmazeutische und/oder kosmetische Verwendung aus der genannten Biomasse.

In besonders vorteilhafter Ausgestaltung ist vorgesehen, dass eine Kahmschicht und/oder auf Wasser aufschwimmende Algenstämme, bspw Fadenalgen der Gattungen Mougeotia oder Gonatozygon, in die Kulturen geimpft wird, welche mit den aquatischen Pflanzen symbiotisch koexistrieren, und das Wachstum der aquatischen Pflanzen begünstigen.

Die besagten Kahmschichten oder Kahmhäute sind als solche bspw aus der Aquaristik bekannt. Hier aber werden die besagten Kahmschichten bewusst geimpft, um den Massenwuchs der aquatischen Pflanzen im genannten erfindungsgemäßen Verfahren mit zu begünstigen.

Es können auch auf Wasser aufschwimmende Algenstämme sein, die das Biomassewachstum der genannten Pflanzen günstig beeinflusst.

Eine weitere Ausgestaltung besteht darin, dass Eisenbakterien und/oder Phytoplankten und/oder phototrophe Bakterien in die Kulturen geimpft werden.

Zu den Eisenbakterien zählen:

- Thiobacillus ferroxydans
- Chlamydobakterien, bspw Leptohrix ochracea
- Cleptohrix polyspora
- Lieskeella bifida
- Gallionella ferriginea
- Siderocapsa treubii
- Hyphomicrobium und Pedomicrobium
- Leptothrix discophora

Diese Bakterien begünstigen nicht nur den photosynthetischen Wirkungsgrad, sondern auch den bspw für Biogas vorteilhafte Einbau von Schwefel und Eisen, und ggfs anderen Spurenelemente. In geringen aber vorhandenen Konzentrationen könne diese aber auch für die Verwendung als Futtermittel vorteilhaft sein:

Hinzu kommen auch alle Phytoplankten, sowie phototrophe Bakterien.

Diese Bakterien begünstigen den Sachverhalt, dass biochemische Reaktionen zur Verstoffwechselung von $CO_2$ zu Kohlenhydraten auch in lichtarmen Bereichen der Kultur ablaufen kann.

Die ebenso genannten aquatischen Biomassepflanzen sind auf Eignung als Schwachlichtpflanzen selektiert. In Koexistenz mit den besagten Eisenbakterien ist deren Stoffwechsel katalytisch angeregt.

In Bezug auf die Inhaltsstoffe ergibt sich für genannte spezifische Biomasse folgendes im Überblick:

|  | Rohfett | Protein | Stärke | RNB | Lakt. | ME |
|---|---|---|---|---|---|---|
| Lemna | 0,9-5 | 15-26 | 17-22 | 9,0-15 | Ca 6,0 | 9,9 |
| Azolla | 4,1 | 30,7 | 3,4 | 20,4 | 6,3 | 10,4 |
| Eichhornia | 1,7 | 22,9 | 6,9 | 13,2 | 5,31 | 8,8 |
| Silvana | 2,0 | 28,2 | 3,1 | 18,3 | 5,83 | 9,6 |
| Pistia | 2,2 | 19,7 | 16 | 7,5 | 5,94 | 9,8 |

Die Angaben sind in % von der Trockenmasse.

Die Einzelbestimmungen erfolgten unter:

ME (Rind), sowie Netto-Energie-Laktation NEL (Lakt.) sowie RNB-Wert (Ruminale N-Bilanz) sind unter der Methode DLG-Formel ermittelt nach der LUFA NRW.

Ferner ist antibiotische Aktivität nach der Methode Plattendiffusionstest (4 Platten) nicht nachweisbar, d.h. dass diese nicht vorhanden ist.

Alle diese Werte legen somit eine erhebliche Eignung als Futtermittel dar. Hinzu kommt, dass die genannten Pflanzen gegenüber Algen deutlich leichter zu generieren sind, und deutlich mehr Biomasse pro Zeiteinheit produzieren, und im Speziellen die Lemna große Mengen $CO_2$ aufnehmen und verstoffwechseln.

Zusätzlich sind zu den genannten Pflanzen Gasausbeuten ermittelt worden, die wie folgt lauten.

Pistia: 571,33 liter/kg OTS
(OTS= Organisch Trockensubstanz)

Lemna: 551,81 bis 586,61 liter/kg OTS

Silvana: 542,17 liter/kg OTS

Azolla: 543,73 liter/kg OTS

Eichhornia: 555,31 liter/kg OTS

Damit steht fest, dass die Eignung als Futtermittelgrundstoff ebenso gegeben ist wie die Biogaseignung. Damit weisen diese genannten Pflanzen eine erhebliche vorteilhafte Nutzung im Sinne eines Mehrfachnutzens auf. Die erfindungsgemäße Vermehrung der Biomasse resultiert hiermit in einen erheblichen und mehrfachen Nutzen in erheblichen Mengen.
Weitere chemische Inhaltsstoffe sind:

- Calcium und Kalium
- Leucin
- Isoleucin
- Lysin
- Valin
- Phenylalanin.

Der Verfahrensbetrieb und der Mehrfachnutzen ist in der Zeichnung dargestellt. Es zeigt:

Figur 1: Produktionsflächen und Entnahme

Figur 2: Mehrfachnutzen

Figur 3: Logistische Einbindung von Kultruflächen und Viehzuchtanlage

Figur 4: Hauptreaktor mit Nachreaktor

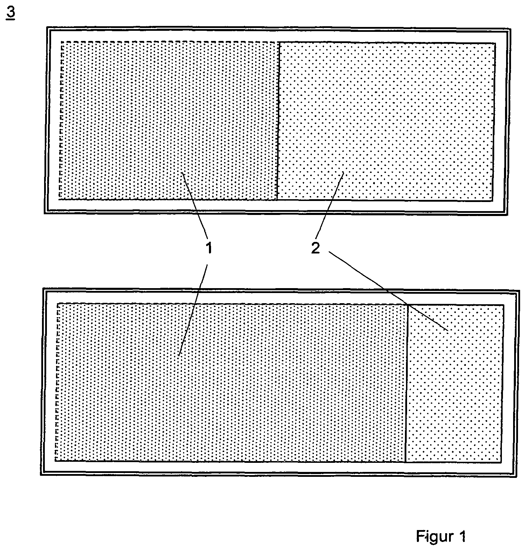

Figur 1 zeigt ein prinzipielle Darstellung der Produktionsflächen in Form eine flachen Wanne 3 in Draufsicht, für die genannten aquatischen Pflanzen. Ein erstes Beispiel im oberen Bildteil eine ganzflächige Bedeckung mit Lemna oder einer oder mehrerer der genannten aquatischen Pflanzen.
Geimpft wird die fläche mit nur wenigen aquatischen Pflanzen. Nach einem exponetiellen Wachstum nach wenigen Wochen ist die gesamte Fläche, ausgehend von nur einer kleinen Startmasse völlig bedeckt.
Hier ist die gesamt bedeckte Fläche in zwei virtuelle Teilfächen 1 und 2 unterteilt. In einer Ausgestaltung entnimmt man immer nur die Hälfte der Fläche, und bis zur nächsten Ernte brauchen die verbleibenden Pflanzen nur einen Vermehrungs- bzw Verdopplungszyklus, der bspw bei Lemna nur noch 3 bis 5 Tage oder sogar weniger beträgt, bis die Fläche wieder ganz voll ist, und man wieder die Hälfte entnehmen kann usw.
Im unteren Bildteil kürzt man die ansonsten oben gegebene Erntezeiten alle 3 Tage herunter auf eine tägliche Ernte, indem man nur weniger als die Hälfte entnimmt, also genau so viel, wie sich in dieser Wanne 3 wieder an einem Tag nachbildet.
In beiden Fällen ist die verbleibende Masse nach der Ernte natürlich größer als die Startmenge, die nur bei ca 1 bis 5 % der Gesamtfläche lag.
Neben der Kultivierung und Beerntung eine Wasseroberfläche können die Kulturen natürlich auch auf feuchten Fließen oder dünnen Scheiben vermehrt werden.
Die Beerntungsweise in bezug auf die Aufrechterhaltung eine masseoptimierten vermehrung bleibt aber die gleiche wie in Figur 1 dargstellt.

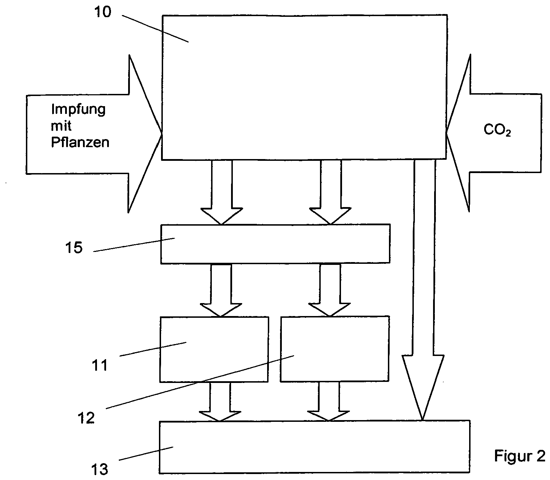

Figur 2 zeigt den Verwertungsfluss der Biomasse. Die Wannen oder Rinnen 3 sind in einem geschlossenen Gewächshaus 10 angesetzt, welches zur Wuchsförderung mit $CO_2$ gespeist wird. Dies kann $CO_2$ aus $CO_2$-haltigem Abgas von Verbrennungsanlagen, Biogasaufmethanisierungsanlagen, Bioenthanolanlagen, chemischen Produktionsanlagen etc sein, welches gegebenenfalls zuvor durch einen Gaswäscher lief, um Schadstoffe zu entfernen.
Die Kulturfläche werden dann mit den genannten Pflanzen geimpft. Optional kann zur Impfung mit den genannten Pflanzen entweder sofort oder zu einem späteren Zeitpunkt die genannten Kahmschichten auch geimpft werden. Optional wird die entnommene Biomasse zunächst in einer Trocknung 15 getrocknet und dann der Futtermittel- oder Futtermittelgrundstoffproduktion 11 zugeführt. Alternativ oder auch ergänzend kann in Bezug auf eine ggfs Aufteilung der Biomasse auch eine chemische Auswertung 12 der Biomasse durch Auszugsverfahren stattfinden, in denen chemische Grundstoffe gewonnen werden.

Die Reststoffe aus ggfs beiden Nutzungen 11 und 12 können dabei noch einer weiteren Nutzung in einer Biogas- oder Bioethanolanlage 13 zugeführt werden.

Hierfür sprechen die oben dargestellten, als hervorragend erzielten Biogaserträge dieser speziellen Biomasse aus den genannten aquatischen Pflanzen.

Alternativ zur Resteverwertung aus Chemie und Futtermittel kann aber die Biomasse natürlich auch direkt der Biogas- und/oder Bioethanolerzeugung 13 zugeführt werden.

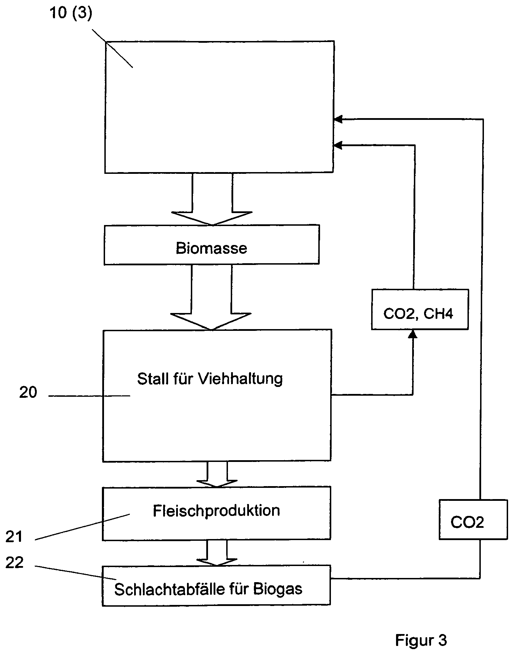

Figur 3 zeigt ein Ausgestaltungsbeispiel, bei welchem, die Kulturflächen 3 bzw das Gewächshaus 10, in welchem diese platziert sind, die erzeugte Biomasse in einen in nächster Näher angelegten Viehstall 20 (Rinder, Schweine, Hühner etc) als Futtermittel eingebracht werden. Dabei kann die Biomasse zuvor auch noch als Futtermittel aufbereitet werden.

Die daran gebundene Fleischproduktion liefert wiederum Schlachtabfälle, die biogastauglich sind. Ferner entsteht sowohl bei der Tierhaltung als auch bei der Biogasproduktion CO2 welcher in diesem Falle wiederum wertvoller Düngestoff für die aquatischen Pflanzen ist. Auch Dung kann dabei zurückgeführt werden, und dient den aquatischen Pflanzen als Düngestoff.

Bei der Tierhaltung entsteht außerdem Methan welches entweder in das Gewächshaus oder auch der Biogasanlage zugeführt wird.

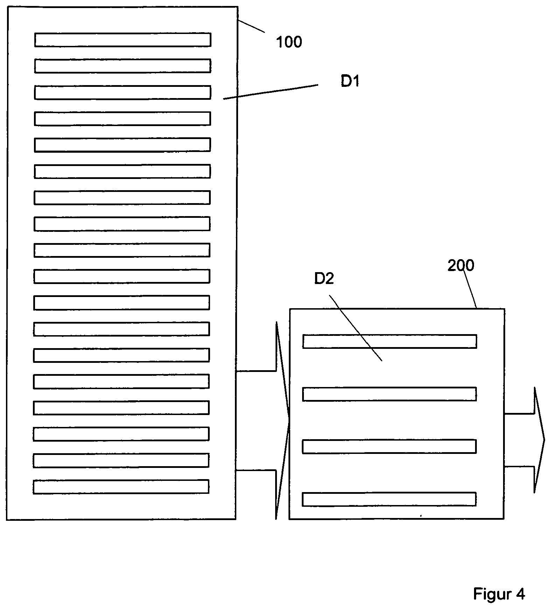

Figur 4 zeigt ein Beispiel für einen Nachreaktor 200 in Verbindung mit einem Hauptreaktor 100.

So ist der Hauptreaktor 100 mit einer vorgegebenen Etagen- oder Stellagendichte D1 von aquatischen Kulturflächen ausgestattet. Dort liegt eine sozusagen in der Wirkung erzielte Lichtreduktion vor, die die gewünschten und durch die Züchtung besonders ausgeprägten natürlichen Schwachlichtbedingungen nachbilden.

Von den dortigen Kulturflächen wird in einem Zyklus von 1 bis 3 Tagen periodisch eine Teilerntung im erfindungsgemäßen Sinne von weniger als 50% vorgenommen. In der Praxis haben sich dauerhaft 20% täglich als für die Population realisierbar ergeben.

Ist nun im Hauptreaktor 100 eine Etagenanordnung von bspw 45 Etagen Aquakulturflächen gewählt, so braucht der Nachreaktor 200 nur noch eine Etagendichte von 9 Etagen oder weniger, wenn bspw im Nachreaktor unter Starklichtbedingungen täglich jeweils 100 % geerntet wird, aber aus dem Hauptreaktor eine geringere Erntemenge als 20% täglich wieder auf die Kulturflächen des Nachreaktors zur Nachverdopplung überführt werden.

Es ist auch möglich, dass der Nachreaktor 200 aus einer Licht ausgesetzten Transporteinrichtung besteht, in der die Biomasse so langsam aber dafür kontinuierlich bewegt wird, dass sich auf dem Transportweg vom Reaktor zur Verwertung ebenfalls ein wie oben beschriebene mindestens weitere finale Verdopplung der aquatischen Pflanzen ergibt. Somit wäre eine solche Transporteinrichtung als ein Nach"vermehrungs"reaktor anzusehen.

## Patentansprüche

1. Verfahren zur Erzeugung von chemischen und/oder pharmazeutischen und/oder kosmetischen Grundstoffen und/oder Futtermittelgrundstoffen und/oder Nahrungsmittelgrundstoffen aus der Biomasse aquatischer Pflanzen, wobei die aquatischen Pflanzen selbstvermehrende aquatische Pflanzen sind, und ausgehend von einer Startmenge zunächst in einer Kultur vermehrt, und sodann in regelmäßigen Zeitabständen geerntet wird, **dadurch gekennzeichnet, dass** zur Erzielung großer Mengen Biomasse, die Wasserflächen/Kulturflächen in einem geschlossenen Gewächshaussystem in aufgestapelten Stellagen, bei denen sich eine effektive Beleuchtungsstärke (L1) von Wasserlinsen unter Schwachlichtbedingungen ergibt, welche in mit $CO_2$ aus einer $CO_2$-Quelle gespeisten Wasserrinnen oder Wannen angelegt sind, und dass ausgehend von einer besagten Startmenge die Vermehrung bis zum Erreichen einer Zielmenge oder Zielfläche der Kultur abgewartet wird, und eine Beerntung entweder bis zum halben Flächenmaß erfolgt, und bis zur nächsten Beerntung wieder auf bis zu dem halben Flächen- oder Gewichtsmaß durch Beerntung reduziert wird, oder dass bei täglicher Beerntung weniger als die Hälfte entnommen wird, und zwar genau soviel, wie sich in den Wannen an einem Tag wieder nachbildet.

2. Verfahren nach Anspruch 1,
   **dadurch gekennzeichnet,**
   **dass** nach Erreichen einer vollen Zielfläche eine tägliche Beerntung in dem Umfang durchgeführt wird, dass die beerntete Erntemenge gerade so groß ist, dass die Pflanzen wieder innerhalb eines Tages die volle Zielfläche erreichen/bedecken.

3. Verfahren nach Anspruch 1 oder 2,
   **dadurch gekennzeichnet,**

**dass** die gesamten aquatischen Kulturflächen sich in mindestens zwei Bioreaktoren verteilen, und zwar in der Art, dass in einem als Hauptreaktor bezeichneten ersten Reaktor eine vorgegebene erste Stellagen-Etagendichte (D1) für die übereinander gestapelten aquatischen Kulturen vorgesehen ist, derart, dass sich eine effektive Beleuchtungsstärke (L1) der aquatischen Flächen unter Schwachlichtbedingungen ergibt, und die Biomasse dort auf die besagte Weise vermehrt, und dass die dort periodisch erntbare Biomasse von dem Hauptreaktor in einen Nachreaktor für einen oder wenige Tage transferiert wird, wobei im Nachreaktor eine effektive Beleuchtungs- oder Ausleuchtungsstärke durch eine geringere Etagendichte D2, mit D2 kleiner als D1, eingestellt ist, in der die dort hinein transferierten aquatischen Pflanzen noch eine weitere Massenvermehrung unter Starklichtbedingungen erhalten, bevor sie final geerntet werden.

4. Verfahren nach einem der vorhergehenden Ansprüche,
**dadurch gekennzeichnet,**
**dass** nach Erreichen einer vollen Zielfläche eine tägliche Beerntung in dem Umfang durchgeführt wird, dass die geerntete Erntemenge gerade so groß ist, dass die verbleibenden Pflanzen wieder innerhalb eines Tages die volle Zielfläche erreichen/bedecken.

5. Verfahren nach einem der Ansprüche 1 bis 4,
**dadurch gekennzeichnet,**
**dass** die chemischen und/oder pharmazeutischen und/oder kosmetischen Grundstoffe und/oder Futter-oder Nahrungsmittelgrundstoffe aus der Biomasse der Wasserlinse Lemna, im Besonderen der Sorte (CPVO Nr 2009/0984) Lemna Minor L Henry Blanke,
und/oder aus der Biomasse der Eichhornia Crassipes, im Besonderen der Sorte (CPVO Nr 2009/0985) Eichhornia Johann Schoofs Sen,
und/oder der Biomasse der Azolla, im Besonderen der Sorte (CPVO Nr 2009/0986) Azolla Avantii, und/oder aus der Biomasse einer Pflanze aus der Familie der Pistia und/oder der Silvana und/oder der Elodea, und/oder aus der Biomasse einer Pflanze aus der Spirodela polyrhiza und/oder der Nastrium officinale und/oder der Ipomea aquatica,
und/oder
aus der Biomasse einer Pflanze aus der Spirodela polyrhiza gewonnen wird.

6. Verfahren nach einem der vorhergehenden Ansprüche,
**dadurch gekennzeichnet,**
**dass** mehrere Pflanzen in Mischkultur kultiviert/produziert oder vermehrt werden.

7. Verfahren nach einem der vorhergehenden Ansprüche,
**dadurch gekennzeichnet,**
**dass** eine Kahmschicht und/oder auf Wasser aufschwimmende Algenstämme, nämlich Fadenalgen der Gattungen Mougeotia oder Gonatozagon, in die Kulturen geimpft wird, welche mit den aquatischen Pflanzen symbiotisch koexistieren, und das Wachstum der aquatischen Pflanzen begünstigen.

8. Verfahren nach Anspruch 7,
**dadurch gekennzeichnet,**
**dass** Eisenbakterien und/oder Phytoplankten und/oder phototrophe Bakterien in die Kulturen geimpft werden.

9. Verfahren nach einem der vorhergehenden Ansprüche,
**dadurch gekennzeichnet,**
**dass** die Kulturflächen und/oder das besagte Gewächshaus (Bioreaktor) in räumlicher Nähe zu einer Viehzuchtanlage steht, und dass ein Biomassezyklus hergestellt wird, indem erzeugte Biomasse verfüttert, und Dung und/oder $CO_2$ und/oder Methan aus der Viehhaltung in die Kulturflächen bzw das Gewächshaus (Bioreaktor) zurückgespeist werden.

10. Verfahren nach einem der vorhergehenden Ansprüche,
**dadurch gekennzeichnet,**
**dass** im Nachreaktor die Wassertiefe der dortigen aquatischen Kulturflächen größer ist als im Hauptreaktor.

11. Verfahren nach einem der vorhergehenden Ansprüche,
**dadurch gekennzeichnet,**
**dass** im Hauptreaktor die Wassermengen der Kulturflächen häufiger mit Frischwasser oder gedüngtem Wasser gespült werden, als im Nachreaktor.

**Claims**

1.  Method for generating chemical and/or pharmaceutical and/or cosmetic basic materials and/or feedstuff basic materials and/or foodstuff basic materials from the biomass of aquatic plants, wherein the aquatic plants are self-propagating aquatic plants and, starting from a starting quantity, are first propagated in a culture, and then harvested at regular intervals, **characterized in that**, in order to attain large quantities of biomass, the water areas/culture areas are set up in a closed greenhouse system in stacked racks of water-filled troughs or vats supplied with $CO_2$ from a $CO_2$ source, with which an effective illumination intensity (L1) of duckweed under low light conditions results, and that starting from said starting quantity, the propagation interval is waited until a target quantity or target area of the culture is attained, and either up to half of the area is harvested, until the next harvest and again reduced by harvesting to half of the area or weight, or that less than half is removed by daily harvesting, namely exactly as much as can regrow in the vats in a day.

2.  Method according to Claim 1, **characterized in that** after attaining a full target area, a daily harvesting is carried out in which the quantity harvested is no more than the plants are able to replace; i.e., attain/cover the entire target area again, within a day.

3.  Method according to Claim 1 or 2, **characterized in that** all of the aquaculture areas are distributed in at least two bioreactors, namely in such a way that, in a first reactor designated as the main reactor, a prespecified first rack level density (D1) is provided for the aquaculture units stacked above one another in such a way as to give rise to an effective illumination intensity (L1) of the aquatic areas under low light conditions, and the biomass propagates therein in the aforesaid fashion, and that the biomass that can be periodically harvested therein is transferred from the main reactor into a secondary reactor for one or a few days, wherein an effective lighting or illumination intensity is established in the secondary reactor by a lower rack level density D2, with D2 being less than D1, in which the aquatic plants transferred therein undergo a further mass propagation under intense light conditions before they are finally harvested.

4.  Method according to one of the preceding claims, **characterized in that** after attaining a full target area, a daily harvesting is carried out in which the quantity harvested is no more than the remaining plants are able to replace; i.e., attain/cover the entire target area again, within a day.

5.  Method according to one of Claims 1 through 4, **characterized in that** the chemical and/or pharmaceutical and/or cosmetic basic materials and/or feedstuff basic materials or foodstuff basic materials are obtained from the biomass of the duckweed *Lemna,* in particular the variety (CPVO no. 2009/0984) *Lemna minor* L. Henry Blanke, and/or from the biomass of *Eichhornia crassipes,* in particular the variety (CPVO no. 2009/0985) *Eichhornia* Johann Schoofs Sen, and/or from the biomass of *Azolla,* in particular the variety (CPVO no. 2009/0986) *Azolla* 'Avantii', and/or from the biomass of a plant from the family comprising *Pistia* and/or *Silvana* and/or *Elodea,* and/or from the biomass of a plant of *Spirodela polyrhiza* and/or *Nastrium officinale* and/or *Ipomea aquatica,* and/or from the biomass of a plant of *Spirodela polyrhiza.*

6.  Method according to one of the preceding claims, **characterized in that** several plants are grown/produced or propagated in mixed culture.

7.  Method according to one of the preceding claims, **characterized in that** the cultures are inoculated with a biofilm and/or with floating algae strains, namely filamentous algae of the genera *Mougeotia* or *Gonatozagon,* which coexist symbiotically with the aquatic plants and enhance the growth of the aquatic plants.

8.  Method according to Claim 7, **characterized in that** the cultures are inoculated with iron bacteria and/or phytoplankton and/or phototrophic bacteria.

9.  Method as in one of the preceding claims, **characterized in that** the culture areas and/or said greenhouse (bioreactor) is located in physical proximity to a livestock raising facility, and that a biomass cycle is established wherein generated biomass is fed, and dung and/or $CO_2$ and/or methane from the livestock operation are fed back into the culture areas or the greenhouse (bioreactor).

**10.** Method as in one of the preceding claims, **characterized in that** the water depth of the aquaculture areas in the secondary reactor is greater than in the main reactor.

**11.** Method as in one of the preceding claims, **characterized in that** the water volumes of the aquaculture areas are flushed with fresh water or water containing fertilizer more frequently in the main reactor than in the secondary reactor.

**Revendications**

**1.** Procédé de production de matières premières chimiques et/ou pharmaceutiques et/ou cosmétiques et/ou de matières premières d'aliments pour animaux et/ou de matières premières de produits alimentaires à partir de la biomasse de plantes aquatiques, les plantes aquatiques étant des plantes aquatiques autoreproductrices et, à partir d'une quantité de départ, étant d'abord multipliées dans une culture, puis récoltées à intervalles temporels réguliers, **caractérisé en ce que**, pour l'obtention de grandes quantités de biomasse, les surfaces d'eau/surfaces de culture sont disposées dans un système de serre fermé en étagères superposées dans lesquelles une intensité d'éclairage efficace (L1) de lentilles d'eau est obtenue dans des conditions de lumière faible, dans des rigoles d'eau ou des cuves alimentées avec du $CO_2$ à partir d'une source de $CO_2$, et **en ce qu'**à partir d'une quantité de départ définie, la multiplication est attendue jusqu'à l'obtention d'une quantité cible ou d'une surface cible de la culture, et soit une récolte a lieu jusqu'à la moitié de la mesure de la surface, et la mesure de surface ou de poids est réduite de jusqu'à la moitié par récolte jusqu'à la prochaine récolte, soit moins de la moitié est prélevé lors d'une récolte quotidienne, et ce exactement de sorte qu'elle se forme de nouveau dans les cuves après une journée.

**2.** Procédé selon la revendication 1, **caractérisé en ce qu'**après avoir atteint une surface cible complète, une récolte quotidienne est réalisée dans la mesure où la quantité récoltée est d'une taille telle que les plantes atteignent/recouvrent de nouveau la surface cible complète en une journée.

**3.** Procédé selon la revendication 1 ou 2, **caractérisé en ce que** l'ensemble des surfaces de culture aquatique sont réparties dans au moins deux bioréacteurs, et ce de sorte qu'une première densité d'étagères donnée (D1) pour les cultures aquatiques superposées soit prévue dans un premier réacteur nommé réacteur principal, de manière à obtenir une intensité d'éclairage (L1) efficace des surfaces aquatiques dans des conditions de lumière faible, et la biomasse s'y multiplie de la manière indiquée, et que la biomasse périodiquement récoltable soit transférée depuis le réacteur principal dans un réacteur secondaire pendant un jour ou moins, une intensité d'éclairage ou d'illumination efficace dans le réacteur secondaire étant ajustée par une densité d'étages D2 plus faible, avec D2 inférieure à D1, dans lequel les plantes aquatiques qui y sont transférées reçoivent encore une multiplication en masse supplémentaire dans des conditions de lumière forte avant d'être finalement récoltées.

**4.** Procédé selon l'une quelconque des revendications précédentes, **caractérisé en ce qu'**après avoir atteint une surface cible complète, une récolte quotidienne est réalisée dans la mesure où la quantité récoltée est d'une taille telle que les plantes restantes atteignent/recouvrent de nouveau la surface cible complète en une journée.

**5.** Procédé selon l'une quelconque des revendications 1 à 4, **caractérisé en ce que** les matières premières chimiques et/ou pharmaceutiques et/ou cosmétiques et/ou de matières premières d'aliments pour animaux ou de produits alimentaires sont obtenues à partir de la biomasse de la lentille d'eau Lemna, notamment de la variété (n° OCW 2009/0984) Lemna Minor L Henry Blanke,
et/ou à partir de la biomasse d'Eichhornia Crassipes, notamment de la variété (n° OCW 2009/0985) Eichhornia Johann Schoofs Sen,
et/ou à partir de la biomasse d'Azolla, notamment de la variété (n° OCW 2009/0986) Azolla Avantii,
et/ou
à partir de la biomasse d'une plante de la famille Pistia et/ou Silvana et/ou Elodea,
et/ou
à partir de la biomasse d'une plante de l'espèce Spirodela polyrhiza et/ou Nastrium officinale et/ou Ipomea aquatica,
et/ou
à partir de la biomasse d'une plante de l'espèce Spirodela polyrhiza.

**6.** Procédé selon l'une quelconque des revendications précédentes, **caractérisé en ce que** plusieurs plantes sont cultivées/produites ou multipliées en culture mixte.

**7.** Procédé selon l'une quelconque des revendications précédentes, **caractérisé en ce qu'**une couche de moisissure et/ou des tiges d'algues flottant sur l'eau, à savoir des algues filaires du genre Mougeotia ou Gonatozagon, sont inoculées dans les cultures, qui coexistent symbiotiquement avec les plantes aquatiques et favorisent la croissance des plantes aquatiques.

8. Procédé selon la revendication 7, **caractérisé en ce que** des bactéries du fer et/ou du phytoplancton et/ou des bactéries phototrophes sont inoculées dans les cultures.

9. Procédé selon l'une quelconque des revendications précédentes, **caractérisé en ce que** les surfaces de culture et/ou ladite serre (bioréacteur) sont à proximité dans l'espace d'une unité d'élevage, et **en ce qu'**un cycle de biomasse est créé, dans lequel la biomasse produite est utilisée pour l'alimentation, et du fumier et/ou du $CO_2$ et/ou du méthane est réintroduit depuis l'élevage dans les surfaces de culture ou la serre (bioréacteur).

10. Procédé selon l'une quelconque des revendications précédentes, **caractérisé en ce que** la profondeur d'eau des surfaces de culture aquatique dans le réacteur secondaire est supérieure à celle dans le réacteur principal.

11. Procédé selon l'une quelconque des revendications précédentes, **caractérisé en ce que** les quantités d'eau des surfaces de culture sont rincées avec de l'eau fraîche ou de l'eau fertilisée plus souvent dans le réacteur principal que dans le réacteur secondaire.

Figur 1

10

Impfung
mit
Pflanzen

CO$_2$

15

11

12

13

Figur 2

10 (3)

Biomasse

CO2, CH4

Stall für Viehhaltung

20

Fleischproduktion

21

CO2

22

Schlachtabfälle für Biogas

Figur 3

100

D1

D2

200

Figur 4

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Nicht-Patentliteratur**

- *Biosource Technology,* 1992, vol. 41, 213-216

- **P.R. BALASUBRAMANIAN ; KASTURI BAI.** *sowie aus dem Aufsatz aus Clean,* 2009, vol. 37 (1), 17-25